# EUROPEAN PATENT APPLICATION

(11) **EP 4 049 662 A1**
(43) Date of publication of application: **31.08.2022**
(21) Application number: 20879398.4
(22) Date of filing: 21.10.2020
(51) Int. Cl.: A61K 31/519, A61K 31/53, A61K 31/131, A61K 31/4458, A61K 45/06, A61P 25/28, C07D 487/04

(54) **USE OF IMIDAZOPYRIMIDINE OR IMIDAZOTRIAZINE COMPOUNDS FOR PREVENTION, ALLEVIATION, OR TREATMENT OF COGNITIVE DISORDERS, OR FOR IMPROVING COGNITIVE FUNCTION**

(30) Priority: 21.10.2019 KR 20190130384
(71) Applicant: SK Biopharmaceuticals Co., Ltd., Seongnam-si, Gyeonggi-do 13494 (KR)
(72) Inventor: JOUNG, Chan Mi, Seongnam-si Gyeonggi-do 13494 (KR); CHUNG, Jin Yong, Seongnam-si Gyeonggi-do 13494 (KR)
(74) Representative: Vossius & Partner Patentanwälte Rechtsanwälte mbB
(86) International application number: PCT/KR2020/014399
(87) International publication number: WO 2021/080312

(57) **Abstract**

The present invention relates to a use of imidazopyrimidine or imidazotriazine compounds of Chemical Formula 1, or pharmaceutically acceptable salts, solvates, or hydrates thereof, for preventing, alleviating or treating cognitive disorder or for improving enhancing cognitive function.

## Description

### [Technical Field]

The present invention relates to a use of an imidazopyrimidine or imidazotriazine compounds of Chemical Formula 1, or pharmaceutically acceptable salts, solvates, or hydrates thereof for preventing, alleviating or treating cognitive disorder, or for improving cognitive function: 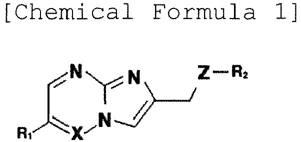 in Chemical Formula 1, X, Z, R₁ and R₂ are as defined herein.

### [Background Art]

The human brain has the ability to acquire, store, and retrieve information over decades. Such collection of information, namely, learning changes the physiological state of specific neurons in the process of encoding a memory. These physiological changes are changes in cell activity caused by learning to be part of the neural code for that memory. These changes may occur through the expression or function of ion channels that excite neurons more or less. This may result in an increase or decrease in the action potential, or may appear more indirectly through changes in other electrical signals. Learning may proceed with the process of nerve growth through new connections or, conversely, the process of neural withdrawal to remove existing connections. Learning may also proceed with the process of cell signal adaptation through changes in the overall ability of neurons to integrate different types of signals, or may also bring about morphological and functional changes in synapses that increase or decrease the ability of neurons. Molecular and cellular memory engrams produced by all neurons involved in this learning act as guides that enable retrieval of memory information later. After the "index" memory engram cells are encoded, the interconnection of cells connected between concepts is strengthened and logically efficiently managed through a simplification process according to perceptual concepts. However, the number of memory engrams continues to accumulate as time passes. Therefore, through the process of forgetting to delete unused or infrequently used memory engrams, the efficiency of brain capacity is further increased. All processes of creating and managing memory engrams so that information about previously experienced events or contents can be recalled and recognized later is a cognitive process (Davis & Zhong, 2017, "The Biology of Forgetting-A Perspective". Neuron. Aug 2;95(3):490-503).

Improving cognitive function means that the activities of cells are made in the direction in which new connections are well formed in the process of nerve growth, the process of cell signal adaptation is consolidated, or the interconnection of cells connected between concepts is more logical in the acquisition, storage, and retrieval of information. Alternatively, improving cognitive function is to prevent cognitive impairment in the direction in which the necessary memory engrams are lost by the process of forgetting, namely, the natural decline of the organism, a similar memory engram that prevents proper retrieval of the desired information is created, or memory retrieval is difficult due to changes in the structure of memory engrams.

Cognitive disorder is a neurological disorder in which memory, attention, language ability, judgment and the like are deteriorated in the cognitive process, and varies from a very mild condition to a severe condition. Mild cognitive impairment is a condition in which the ability to perform daily activities is preserved and the ability to perform instrumental activities is possible but cognitive function is impaired. Dementia is a condition in which cognitive function is deteriorated so as to interfere with daily life. Mild cognitive impairment may occur as a transitional impairment in the course of onset of dementia. In addition, aging, genetics, cardiovascular disease and the like are considered as risk factors of cognitive disorder.

### [Disclosure of Invention]

### [Technical Problem]

An object of the present invention is to provide a method for prevention, alleviation or treatment of cognitive disorder, or for improving cognitive function.

Another object of the present invention is to provide a use of an imidazopyrimidine or imidazotriazine compound of Chemical Formula 1, or a pharmaceutically acceptable salt, solvate or hydrate thereof for prevention, alleviation or treatment of cognitive disorder, or for improving cognitive function: 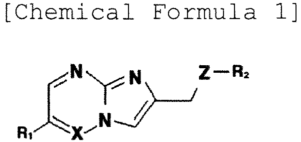 in Chemical Formula 1, X, Z, R₁ and R₂ are as defined herein.

### [Solution to Problem]

The present invention provides a medicament for prevention, alleviation or treatment of cognitive disorder, or for improving cognitive function, which comprises a therapeutically effective amount of an imidazopyrimidine or imidazotriazine compound of Chemical Formula 1, or a pharmaceutically acceptable salt, solvate or hydrate thereof: 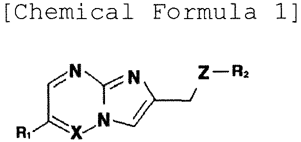 in Chemical Formula 1,
X is CH or N;
Z is O or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-Ci-Cs alkyl, hydroxy-C₁-C₅ alkyl, C₁-C₅ alkoxy-C₁-C₅ alkyl, carbamoyloxy-Ci-Cs alkyl, C₁-C₅ alkyl-C(O)O-C₁-C₅ alkyl, a 5- or 6-membered heterocycloalkyl-C₁-C₅ alkyl having 1 to 3 heteroatoms selected from N, O and S, or di (C₁-C₅ alkyl) amino-C₁-C₅ alkyl; and 5- to 12-membered unsaturated heterocyclyl having 1 to 5 heteroatoms selected from N, O and S, unsubstituted or substituted with one or more substituents selected from halo, hydroxy, C₁-C₅ alkyl, C₁-C₅ alkoxy, and halo-C₁-C₅ alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

The present invention also provides a pharmaceutical composition for prevention, alleviation or treatment of cognitive disorder, or for improving cognitive function, which comprises a therapeutically effective amount of the imidazopyrimidine or imidazotriazine compound of Chemical Formula 1, or a pharmaceutically acceptable salt, solvate or hydrate thereof, and additionally one or more pharmaceutically acceptable carriers.

The present invention also provides a method for prevention, alleviation or treatment of cognitive disorder, or for improving cognitive function, which comprises administering to a subject a therapeutically effective amount of the imidazopyrimidine or imidazotriazine compound of Chemical Formula 1, or a pharmaceutically acceptable salt, solvate or hydrate thereof.

The present invention also provides use of the imidazopyrimidine or imidazotriazine compound of Chemical Formula 1, or a pharmaceutically acceptable salt, solvate or hydrate thereof for prevention, alleviation or treatment of cognitive disorder, or for improving cognitive function.

According to an embodiment of the present invention, R₁ in Chemical Formula 1 is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-Ci-Cs alkyl, carbamoyloxy-C₁-C₅ alkyl and C₁-C₅ alkyl-C(O)O-C₁-C₅ alkyl; or 5- to 10-membered unsaturated heterocyclyl having 1 to 3 heteroatoms selected from N, O and S, unsubstituted or substituted with 1 to 3 substituents selected from halo, hydroxy, C₁-C₅ alkyl, C₁-C₅ alkoxy and halo-C₁-C₅ alkyl.

According to another embodiment of the present invention, R₂ in Chemical Formula 1 is phenyl unsubstituted or substituted with 1 to 5 substituents selected from halo, deuterium, hydroxy and C₁-C₅ alkyl; or 5- or 6-membered heteroaryl having 1 to 3 heteroatoms selected from N, O and S, unsubstituted or substituted with 1 to 3 substituents selected from halo and C₁-C₅ alkyl.

According to another embodiment of the present invention, in Chemical Formula 1,
X is CH or N;
Z is 0;
R₁ is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl and C₁-C₅ alkyl-C(O)O-C₁-C₅ alkyl; or 5- to 9-membered unsaturated heterocyclyl having 1 or 2 heteroatoms selected from N, O and S, unsubstituted or substituted with 1 or 2 substituents selected from halo, hydroxy, C₁-C₅ alkyl, C₁-C₅ alkoxy and halo-C₁-C₅ alkyl; and
R₂ is phenyl unsubstituted or substituted with 1 to 5 substituents selected from halo, deuterium, hydroxy and C₁-C₅ alkyl; or 6-membered heteroaryl having 1 or 2 nitrogen atoms, unsubstituted or substituted with 1 or 2 substituents selected from halo and C₁-C₅ alkyl.

According to another embodiment of the present invention, in Chemical Formula 1,
R₁ is phenyl unsubstituted or substituted with 1 to 3 substituents selected from halo, hydroxy, C₁-C₅ alkyl, C₁-C₅ alkoxy, C₁-C₅ alkylthio, amino, di (C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl and C₁-C₅ alkyl-C(O)O-C₁-C₅ alkyl; 1,3-benzodioxolyl unsubstituted or substituted with 1 or 2 halo; or pyridyl or pyrimidinyl unsubstituted or substituted with 1 or 2 substituents selected from halo, hydroxy, C₁-C₅ alkyl, C₁-C₅ alkoxy and halo-C₁-C₅ alkyl, and
R₂ is phenyl unsubstituted or substituted with 1 to 5 substituents selected from halo, deuterium, hydroxy and C₁-C₅ alkyl; or pyridyl unsubstituted or substituted with 1 or 2 substituents selected from halo and C₁-C₅ alkyl.

According to another embodiment of the present invention, in Chemical Formula 1,
X is CH;
Z is O;
R₁ is phenyl unsubstituted or substituted with 1 to 3 substituents selected from halo, hydroxy, C₁-C₅ alkyl, C₁-C₅ alkoxy, C₁-C₅ alkylthio, amino, halo-C₁-C₅ alkyl, hydroxy-C₁-C₅ alkyl and C₁-C₅ alkoxy-C₁-C₅ alkyl; and
R₂ is pyridyl unsubstituted or substituted with 1 or 2 substituents selected from halo and C₁-C₅ alkyl.

Representatives of the compound of Chemical Formula 1 according to the present invention include:
6-(2-fluorophenyl)-2-phenoxymethylimidazo[1,2-a]pyrimidine;
2-phenoxymethyl-6-phenylimidazo[1,2-a]pyrimidine; 6-(2,4-difluorophenyl)-2-phenoxymethylimidazo[1,2-a]pyrimidine;
6-(2-methoxyphenyl)-2-phenoxymethylimidazo[1,2-a]pyrimidine;
6-(2-methylphenyl)-2-phenoxymethylimidazo[1,2-a]pyrimidine;
6-(4-fluoro-2-methylphenyl)-2-phenoxymethylimidazo[1,2-a]pyrimidine;
6-(2,3-difluorophenyl)-2-phenoxymethylimidazo[1,2-a]pyrimidine;
6-(4-fluorophenyl)-2-phenoxymethylimidazo[1,2-a]pyrimidine;
6-(3-aminophenyl)-2-phenoxymethylimidazo[1,2-a]pyrimidine;
6-(2-aminophenyl)-2-phenoxymethylimidazo[1,2-a]pyrimidine;
6-(3-amino-6-methylphenyl)-2-phenoxymethylimidazo[1,2-a]pyrimidine;
6-(3-amino-4-fluorophenyl)-2-phenoxymethylimidazo[1,2-a]pyrimidine;
6-(3-chloro-4-fluorophenyl)-2-phenoxymethylimidazo[1,2-a]pyrimidine;
6-(2-dimethylaminophenyl)-2-phenoxymethylimidazo[1,2-a]pyrimidine;
6-(2-chloro-4-fluorophenyl)-2-phenoxymethylimidazo[1,2-a]pyrimidine;
6-(2-hydroxyphenyl)-2-phenoxymethylimidazo[1,2-a]pyrimidine;
6-(3-hydroxyphenyl)-2-phenoxymethylimidazo[1,2-a]pyrimidine;
6-(4-fluoro-2-trifluoromethylphenyl)-2-phenoxymethylimidazo[1,2-a]pyrimidine;
6-(3,4-difluorophenyl)-2-phenoxymethylimidazo[1,2-a]pyrimidine;
6-(2-methoxy-4-fluorophenyl)-2-phenoxymethylimidazo[1,2-a]pyrimidine;
6-(4-fluoro-3-methoxyphenyl)-2-phenoxymethylimidazo[1,2-a]pyrimidine;
6-(4-chloro-2-methoxyphenyl)-2-phenoxymethylimidazo[1,2-a]pyrimidine;
6-(4-cyano-2-methoxyphenyl)-2-phenoxymethylimidazo[1,2-a]pyrimidine;
6-(7-fluorobenzo[1,3]dioxol-4-yl)-2-phenoxymethylimidazo[1,2-a]pyrimidine;
6-(4-fluoro-2-hydroxymethylphenyl)-2-phenoxymethylimidazo[1,2-a]pyrimidine;
6-(4-fluoro-2-methylthiophenyl)-2-phenoxymethylimidazo[1,2-a]pyrimidine;
6-(2-amino-4-fluorophenyl)-2-phenoxymethylimidazo[1,2-a]pyrimidine;
6-(2,4-difluoro-5-methoxyphenyl)-2-phenoxymethylimidazo[1,2-a]pyrimidine;
6-(2-fluoropyridin-3-yl)-2-phenoxymethylimidazo[1,2-a]pyrimidine;
6-(6-methoxypyridin-3-yl)-2-phenoxymethylimidazo[1,2-a]pyrimidine;
6-(6-fluoropyridin-3-yl)-2-phenoxymethylimidazo[1,2-a]pyrimidine;
6-(4-methylpyridin-3-yl)-2-phenoxymethylimidazo[1,2-a]pyrimidine;
6-(2,6-difluoropyridin-3-yl)-2-phenoxymethylimidazo[1,2-a]pyrimidine;
6-(6-chloropyridin-3-yl)-2-phenoxymethylimidazo[1,2-a]pyrimidine;
6-(2-fluoropyridin-4-yl)-2-phenoxymethylimidazo[1,2-a]pyrimidine;
6-(3-chloropyridin-4-yl)-2-phenoxymethylimidazo[1,2-a]pyrimidine;
6-(4-chlorophenyl)-2-phenoxymethylimidazo[1,2-a]pyrimidine;
6-(6-fluoro-4-methyl-3-pyridyl)-2-phenoxymethylimidazo[1,2-a]pyrimidine;
6-(6-fluoro-5-methyl-3-pyridyl)-2-phenoxymethylimidazo[1,2-a]pyrimidine;
6-(5-fluoro-2-pyridyl)-2-phenoxymethylimidazo[1,2-a]pyrimidine;
6-(2,4-difluorophenyl)-2-(3-fluorophenoxymethyl)imidazo[1,2-a]pyrimidine;
6-(4-fluoro-2-methoxyphenyl)-2-(3-fluorophenoxymethyl)imidazo[1,2-a]pyrimidine;
6-(4-fluoro-3-hydroxyphenyl)-2-(3-fluorophenoxymethyl)-imidazo[1,2-a]pyrimidine;
6-(2-aminophenyl)-2-(3-fluorophenoxymethyl)imidazo[1,2-a]pyrimidine;
6-(3-chloropyridin-4-yl)-2-(3-fluorophenoxymethyl)imidazo[1,2-a]pyrimidine;
6-(4-methylpyridin-3-yl)-2-(3-fluorophenoxymethyl)imidazo[1,2-a]pyrimidine;
6-(2,4-difluorophenyl)-2-(4-fluorophenoxymethyl)imidazo[1,2-a]pyrimidine;
6-(4-fluoro-2-methylphenyl)-2-(4-fluorophenoxymethyl)imidazo[1,2-a]pyrimidine;
6-(3-chloropyridin-4-yl)-2-(4-fluorophenoxymethyl)imidazo[1,2-a]pyrimidine;
6-(2,6-dimethylphenyl)-2-(4-fluorophenoxymethyl)imidazo[1,2-a]pyrimidine;
2-[(4-fluorophenoxy)methyl]-6-(6-fluoro-3-pyridyl)imidazo[1,2-a]pyrimidine;
4-[[6-(4-fluorophenyl)imidazo[1,2-a]pyrimidin-2-yl]methoxy]phenol;
2-[(4-fluorophenoxy)methyl]-6-(4-fluorophenyl)imidazo[1,2-a]pyrimidine;
2-[(3-fluorophenoxy)methyl]-6-(4-fluorophenyl)imidazo[1,2-a]pyrimidine;
2-fluoro-5-[2-[(4-fluorophenoxy)methyl]imidazo[1,2-a]pyrimidin-6-yl]phenol;
2-[(4-fluorophenoxy)methyl]-6-phenyl-imidazo[1,2-a]pyrimidine;
5-fluoro-2-[2-[(3-fluorophenoxy)methyl]imidazo[1,2-a]pyrimidin-6-yl]phenol;
5-fluoro-2-[2-[(4-fluorophenoxy)methyl]imidazo[1,2-a]pyrimidin-6-yl]phenol;
6-(4-fluorophenyl)-2-[(2,3,4,5,6-pentadeuteriophenoxy)methyl]imidazo[1,2-a]pyrimidine;
2-[(4-fluorophenoxy)methyl]6-(o-tolyl)imidazo[1,2-a]pyrimidine;
6-(5-fluoro-2-methyl-phenyl)-2-[(4-fluorophenoxy)methyl]imidazo[1,2-a]pyrimidine;
2-[(4-fluorophenoxy)methyl]-6-(2-fluoro-4-pyridyl)imidazo[1,2-a]pyrimidine;
2-[2-[(4-fluorophenoxy)methyl]imidazo[1,2-a]pyrimidin-6-yl]phenol;
6-(2-fluoro-4-methyl-phenyl)-2-[(4-fluorophenoxy)methyl]imidazo[1,2-a]pyrimidine;
2-[(4-fluorophenoxy)methyl]-6-[6-(trifluoromethyl)-3-pyridyl]imidazo[1,2-a]pyrimidine;
2-[2-[(4-fluorophenoxy)methyl]imidazo[1,2-a]pyrimidin-6-yl]aniline;
6-(2-chloro-4-fluoro-phenyl)-2-[(4-fluorophenoxy)methyl]imidazo[1,2-a]pyrimidine;
4-[2-[(4-fluorophenoxy)methyl]imidazo[1,2-a]pyrimidin-6-yl]-3-methoxy-benzonitrile;
6-(4-chloro-2-methoxy-phenyl)-2-[(4-fluorophenoxy)methyl]imidazo[1,2-a]pyrimidine;
2-fluoro-5-[2-[(4-fluorophenoxy)methyl]imidazo[1,2-a]pyrimidin-6-yl]aniline;
6-(2,6-difluoro-3-pyridyl)-2-[(3-fluorophenoxy)methyl]imidazo[1,2-a]pyrimidine;
5-[2-[(4-fluorophenoxy)methyl]imidazo[1,2-a]pyrimidin-6-yl]-2-methyl-aniline;
4,5-difluoro-2-[2-[(4-fluorophenoxy)methyl]imidazo[1,2-a]pyrimidin-6-yl]aniline;
2-[2-[(4-fluorophenoxy)methyl]imidazo[1,2-a]pyrimidin-6-yl]-5-methyl-aniline;
5-chloro-2-[2-[(4-fluorophenoxy)methyl]imidazo[1,2-a]pyrimidin-6-yl]aniline;
2-[2-[(4-fluorophenoxy)methyl]imidazo[1,2-a]pyrimidin-6-yl]-4-methyl-aniline;
5-fluoro-2-[2-[(4-fluorophenoxy)methyl]imidazo[1,2-a]pyrimidin-6-yl]aniline;
2-[(4-fluorophenoxy)methyl]-6-(4-methyl-3-pyridyl)imidazo[1,2-a]pyrimidine;
2-[(3-fluorophenoxy)methyl]-6-(2-fluoro-4-pyridyl)imidazo[1,2-a]pyrimidine;
2-[(3-fluorophenoxy)methyl]-6-[6-(trifluoromethyl)-3-pyridyl)imidazo[1,2-a]pyrimidine;
2-[(3-fluorophenoxy)methyl]-6-(6-fluoro-3-pyridyl)imidazo[1,2-a]pyrimidine;
2-[(3-fluorophenoxy)methyl]-6-(2-fluoro-3-pyridyl)imidazo[1,2-a]pyrimidine;
6-(5,6-difluoro-3-pyridyl)-2-[(4-fluorophenoxy)methyl]imidazo[1,2-a]pyrimidine;
5-[2-[(4-fluorophenoxy)methyl]imidazo[1,2-a]pyrimidin-6-yl]-2-methoxy-aniline;
2-[(4-fluorophenoxy)methyl]-6-(5-fluoro-2-pyridyl)imidazo[1,2-a]pyrimidine;
2-[(4-fluorophenoxy)methyl]-6-(5-methoxy-2-pyridyl)imidazo[1,2-a]pyrimidine;
6-(4-chloro-3-pyridyl)-2-[(4-fluorophenoxy)methyl]imidazo[1,2-a]pyrimidine;
6-(5-chloro-3-pyridyl)-2-[(4-fluorophenoxy)methyl]imidazo[1,2-a]pyrimidine;
2-[(4-fluorophenoxy)methyl]-6-(5-methoxy-3-pyridyl)imidazo[1,2-a]pyrimidine;
5-[2-[(4-fluorophenoxy)methyl]imidazo[1,2-a]pyrimidin-6-yl]pyridin-2-ol;
6-(6-fluoro-5-methyl-3-pyridyl)-2-[(4-fluorophenoxy)methyl]imidazo[1,2-a]pyrimidine;
2-[(4-fluorophenoxy)methyl]-6-(6-methyl-3-pyridyl)imidazo[1,2-a]pyrimidine;
2-[(3-fluorophenoxy)methyl]-6-(5-fluoro-2-pyridyl)imidazo[1,2-a]pyrimidine;
2-[(3-fluorophenoxy)methyl]-6-[4-fluoro-2-(trifluoromethyl)phenyl]imidazo[1,2-a]pyrimidine;
4-[2-[(3-fluorophenoxy)methyl]imidazo[1,2-a]pyrimidin-6-yl]-3-methoxy-benzonitrile;
[5-fluoro-2-[2-[(3-fluorophenoxy)methyl]imidazo[1,2-a]pyrimidin-6-yl]phenyl]methanol;
[5-fluoro-2-[2-[(4-fluorophenoxy)methyl]imidazo[1,2-a]pyrimidin-6-yl]phenyl]methanol;
6-(4-fluoro-2-methylsulfanyl-phenyl)-2-[(4-fluorophenoxy)methyl]imidazo[1,2-a]pyrimidine;
2-[(4-fluorophenoxy)methyl]-6-(2-methoxy-4-pyridyl)imidazo[1,2-a]pyrimidine;
2-[2-[(3-fluorophenoxy)methyl]imidazo[1,2-a]pyrimidin-6-yl]-4-methyl-aniline;
5-fluoro-2-[2-[(3-fluorophenoxy)methyl]imidazo[1,2-a]pyrimidin-6-yl]aniline;
6-(4-fluorophenyl)-2-(pyridin-2-yloxymethyl)imidazo[1,2-a]pyrimidine;
6-(2-methylphenyl)-2-(pyridin-2-yloxymethyl)imidazo[1,2-a]pyrimidine;
6-(4-fluoro-2-methoxyphenyl)-2-(pyridin-2-yloxymethyl)imidazo[1,2-a]pyrimidine;
6-(2,4-difluorophenyl)-2-(pyridin-2-yloxymethyl)imidazo[1,2-a]pyrimidine;
6-(4-fluoro-2-methylphenyl)-2-(pyridin-2-yloxymethyl)imidazo[1,2-a]pyrimidine;
6-(2,3-difluorophenyl)-2-(pyridin-2-yloxymethyl)imidazo[1,2-a]pyrimidine;
6-(5-fluoro-2-methylphenyl)-2-(pyridin-2-yloxymethyl)imidazo[1,2-a]pyrimidine;
6-(3-fluoro-2-methylphenyl)-2-(pyridin-2-yloxymethyl)imidazo[1,2-a]pyrimidine;
6-(7-fluoro-2H-benzo[1,3]dioxol-4-yl)-2-(pyridin-2-yloxymethyl)imidazo[1,2-a]pyrimidine;
6-(4-chloro-2-methoxyphenyl)-2-(pyridin-2-yloxymethyl)imidazo[1,2-a]pyrimidine;
6-(3-fluoro-2-methoxy-phenyl)-2-(2-pyridyloxymethyl)imidazo[1,2-a]pyrimidine;
6-[4-fluoro-2-(trifluoromethyl)phenyl]-2-(2-pyridyloxymethyl)imidazo[1,2-a]pyrimidine;
6-(4-fluoro-2-methyl-phenyl)-2-[(5-fluoro-2-pyridyl)oxymethyl)imidazo[1,2-a]pyrimidine;
6-(2-ethylphenyl)-2-(2-pyridyloxymethyl)imidazo[1,2-a]pyrimidine;
6-(4-fluoro-2-methyl-phenyl)-2-[(4-fluoro-2-pyridyl)oxymethyl)imidazo[1,2-a]pyrimidine;
6-(2-fluoro-4-methyl-phenyl)-2-(2-pyridyloxymethyl)imidazo[1,2-a]pyrimidine;
6-(4-fluoro-2-methoxy-phenyl)-2-[(4-fluoro-2-pyridyl)oxymethyl)imidazo[1,2-a]pyrimidine;
2-[(5-fluoro-2-pyridyl)oxymethyl]-6-[4-fluoro-2-(trifluoromethyl)phenyl]imidazo[1,2-a]pyrimidine;
6-(2,4-difluorophenyl)-2-[(4-fluoro-2-pyridyl)oxymethyl)imidazo[1,2-alpyrimidine;
6-(3-fluoro-2-methoxy-phenyl)-2-[(4-fluoro-2-pyridyl)oxymethyl)imidazo[1,2-a]pyrimidine;
6-(2,3-difluorophenyl)-2-[(4-fluoro-2-pyridyl)oxymethyl)imidazo[1,2-a]pyrimidine;
6-(4-fluorophenyl)-2-[(4-fluoro-2-pyridyl)oxymethyl)imidazo[1,2-a]pyrimidine;
6-(3-fluoro-2-methyl-phenyl)-2-[(5-fluoro-2-pyridyl)oxymethyl)imidazo[1,2-a]pyrimidine;
2-[(4-fluoro-2-pyridyl)oxymethyl]-6-[4-fluoro-2-(trifluoromethyl)phenyl]imidazo[1,2-a]pyrimidine;
2-[(5-fluoro-2-pyridyl)oxymethyl]-6-(o-tolyl)imidazo[1,2-a]pyrimidine;
6-(4-chloro-2-methyl-phenyl)-2-[(5-fluoro-2-pyridyl)oxymethyl)imidazo[1,2-a]pyrimidine;
6-(2,4-dimethylphenyl)-2-[(5-fluoro-2-pyridyl)oxymethyl)imidazo[1,2-a]pyrimidine;
6-(4-fluorophenyl)-2-[(5-fluoro-2-pyridyl)oxymethyl)imidazo[1,2-a]pyrimidine; 2-(2-pyridyloxymethyl)-6-[6-(trifluoromethyl)-3-pyridyl]imidazo[1,2-a]pyrimidine;
2-[(5-fluoro-2-pyridyl)oxymethyl]-6-[6-(trifluoromethyl)-3-pyridyl]imidazo[1,2-a]pyrimidine;
6-(5-fluoro-2-pyridyl)-2-(2-pyridyloxymethyl)imidazo[1,2-a]pyrimidine;
2-fluoro-5-[2-[(5-fluoro-2-pyridyl)oxymethyl]imidazo[1,2-a]pyrimidin-6-yl]aniline;
2-fluoro-5-[2-(2-pyridyloxymethyl)imidazo[1,2-a]pyrimidin-6-yl]aniline;
[5-fluoro-2-[2-(2-pyridyloxymethyl)imidazo[1,2-a]pyrimidin-6-yl]phenyl]methanol;
3-methoxy-4-[2-(2-pyridyloxymethyl)imidazo[1,2-a]pyrimidin-6-yl]benzonitrile;
4-[2-[(5-fluoro-2-pyridyl)oxymethyl]imidazo[1,2-a]pyrimidin-6-yl]-3-methoxy-benzonitrile;
6-(4-fluoro-2-methylsulfanyl-phenyl)-2-(2-pyridyloxymethyl)imidazo[1,2-a]pyrimidine;
[5-fluoro-2-[2-[(5-fluoro-2-pyridyl)oxymethyl]imidazo[1,2-a]pyrimidin-6-yl]phenyl]methanol;
4-[2-(2-pyridyloxymethyl)imidazo[1,2-a]pyrimidin-6-yl]benzonitrile;
6-[4-fluoro-2-(methoxymethyl)phenyl]-2-(2-pyridyloxymethyl)imidazo[1,2-a]pyrimidine;
[2-[2-(2-pyridyloxymethyl)imidazo[1,2-a]pyrimidin-6-yl]-5-(trifluoromethyl)phenyl]methanol;
6-(2-isopropylphenyl)-2-(2-pyridyloxymethyl)imidazo[1,2-a]pyrimidine;
4-[2-(2-pyridyloxymethyl)imidazo[1,2-a]pyrimidin-6-yl]-3-(trifluoromethyl)benzaldehyde;
6-[4-chloro-2-(trifluoromethyl)phenyl]-2-(2-pyridyloxymethyl)imidazo[1,2-a]pyrimidine;
6-(4-fluorophenyl)-2-(pyridin-3-yloxymethyl)imidazo[1,2-a]pyrimidine;
6-(4-fluoro-2-methoxyphenyl)-2-(pyridin-3-yloxymethyl)imidazo[1,2-a]pyrimidine;
6-(2,4-difluorophenyl)-2-(pyridin-3-yloxymethyl)imidazo[1,2-a]pyrimidine;
6-(4-fluoro-2-methylphenyl)-2-(pyridin-3-yloxymethyl)imidazo[1,2-a]pyrimidine;
6-(4-fluoro-2-hydroxyphenyl)-2-(pyridin-3-yloxymethyl)imidazo[1,2-a]pyrimidine;
6-(4-fluoro-2-methoxyphenyl)-2-(pyridin-4-yloxymethyl)imidazo[1,2-a]pyrimidine;
6-(2,4-difluorophenyl)-2-(pyridin-4-yloxymethyl)imidazo[1,2-a]pyrimidine;
6-(2-methylphenyl)-2-(5-fluoro-pyridin-3-yloxymethyl)imidazo[1,2-a]pyrimidine;
6-(4-fluoro-2-methylphenyl)-2-(5-fluoro-pyridin-3-yloxymethyl)imidazo[1,2-a]pyrimidine;
6-(4-fluoro-2-methoxyphenyl)-2-(5-fluoropyridin-3-yloxymethyl)imidazo[1,2-a]pyrimidine;
6-(4-fluoro-2-hydroxyphenyl)-2-(5-fluoropyridin-3-yloxymethyl)imidazo[1,2-a]pyrimidine;
6-(2,4-difluorophenyl)-2-(6-fluoropyridin-3-yloxymethyl)imidazo[1,2-a]pyrimidine;
6-(4-fluoro-2-methylphenyl)-2-(6-fluoropyridin-3-yloxymethyl)imidazo[1,2-a]pyrimidine;
6-(4-fluoro-2-methylphenyl)-2-(2-fluoropyridin-3-yloxymethyl)imidazo[1,2-a]pyrimidine;
6-(4-fluoro-2-methylphenyl)-2-(5-chloropyridin-3-yloxymethyl)imidazo[1,2-a]pyrimidine;
6-(2,4-difluorophenyl)-2-(2-fluoropyridin-4-yloxymethyl)imidazo[1,2-a]pyrimidine;
6-(4-fluoro-2-methylphenyl)-2-(2-fluoropyridin-4-yloxymethyl)imidazo[1,2-a]pyrimidine;
6-(4-fluoro-2-methoxyphenyl)-2-(2-fluoropyridin-4-yloxymethyl)imidazo[1,2-a]pyrimidine;
6-(4-fluorophenyl)-2-(2-fluoropyridin-4-yloxymethyl)imidazo[1,2-a]pyrimidine;
6-(2,3-difluorophenyl)-2-(2-fluoropyridin-4-yloxymethyl)imidazo[1,2-a]pyrimidine;
6-(2-fluorophenyl)-2-(2-fluoropyridin-4-yloxymethyl)imidazo[1,2-a]pyrimidine;
6-(4-fluoro-2-hydroxyphenyl)-2-(2-fluoropyridin-4-yloxymethyl)imidazo[1,2-a]pyrimidine;
6-(2-methylphenyl)-2-(2-fluoropyridin-4-yloxymethyl)imidazo[1,2-a]pyrimidine;
6-(2-hydroxyphenyl)-2-(2-fluoropyridin-4-yloxymethyl)imidazo[1,2-a]pyrimidine;
6-(4-fluoro-2-hydroxymethylphenyl)-2-(2-fluoropyridin-4-yloxymethyl)imidazo[1,2-a]pyrimidine;
6-(4-fluorophenyl)-2-[(5-fluoro-3-pyridyl)oxymethyl)imidazo[1,2-a]pyrimidine;
2-[(2-chloro-4-pyridyl)oxymethyl]-6-(4-fluorophenyl)imidazo[1,2-a]pyrimidine;
2-[(5-fluoro-3-pyridyl)oxymethyl]-6-[4-fluoro-2-(trifluoromethyl)phenyl]imidazo[1,2-a]pyrimidine;
2-[(2-fluoro-4-pyridyl)oxymethyl]-6-[4-fluoro-2-(trifluoromethyl)phenyl]imidazo[1,2-a]pyrimidine;
5-fluoro-2-[2-[(5-fluoro-3-pyridyl)oxymethyl]imidazo[1,2-a]pyrimidin-6-yl]aniline;
5-fluoro-2-[2-[(2-fluoro-4-pyridyl)oxymethyl]imidazo[1,2-a]pyrimidin-6-yl]aniline;
[5-fluoro-2-[2-[(5-fluoro-3-pyridyl)oxymethyl]imidazo[1,2-a]pyrimidin-6-yl]phenyl]methanol;
2-(2,4-difluorophenyl)-6-(phenoxymethyl)imidazo[1,2-b][1,2,4]triazine;
2-(2,4-difluorophenyl)-6-((pyridin-4-yloxy)methyl)imidazo[1,2-b][1,2,4]triazine;
2-(2,4-difluorophenyl)-6-((pyridin-2-yloxy)methyl)imidazo[1,2-b][1,2,4]triazine;
2-(4-fluorophenyl)-6-((pyridin-4-yloxy)methyl)imidazo[1,2-b][1,2,4]triazine;
2-(2-methylphenyl)-6-((pyridin-4-yloxy)methyl)imidazo[1,2-b][1,2,4]triazine;
2-(2,4-difluorophenyl)-6-((2-fluoropyridin-4-yloxy)methyl)imidazo[1,2-b][1,2,4]triazine;
2-(4-fluoro-2-methylphenyl)-6-((2-fluoropyridin-4-yloxy)methyl)imidazo[1,2-b][1,2,4]triazine;
2-(2-methylphenyl)-6-((2-fluoropyridin-4-yloxy)methyl)imidazo[1,2-b][1,2,4]triazine;
2-[4-fluoro-2-(trifluoromethyl)phenyl]-6-(2-pyridyloxymethyl)imidazo[1,2-b][1,2,4]triazine;
2-(3-fluoro-2-methyl-phenyl)-6-(2-pyridyloxymethyl)imidazo[1,2-b][1,2,4]triazine;
2-(4-fluoro-2-methyl-phenyl)-6-(2-pyridyloxymethyl)imidazo[1,2-b][1,2,4]triazine;
2-[4-chloro-2-(trifluoromethyl)phenyl]-6-(2-pyridyloxymethyl)imidazo[1,2-b][1,2,4]triazine;
2-(4-chloro-2-methyl-phenyl)-6-(2-pyridyloxymethyl)imidazo[1,2-b][1,2,4]triazine;
2-(4-fluoro-2-methyl-phenyl)-6-(2-pyridyloxymethyl)imidazo[1,2-b][1,2,4]triazine;
2-(4-fluoro-2-methyl-phenyl)-6-[(5-fluoro-2-pyridyl)oxymethyl]imidazo[1,2-b][1,2,4]triazine ;
[5-fluoro-2-[2-[(4-fluorophenoxy)methyl]imidazo[1,2-a]pyrimidin-6-yl]phenyl]methyl carbamate;
[5-fluoro-2-[2-(phenoxymethyl)imidazo[1,2-a]pyrimidin-6-yl]phenyl]methyl carbamate;
[5-fluoro-2-[2-(2-pyridyloxymethyl)imidazo[1,2-a]pyrimidin-6-yl]phenyl]methyl carbamate;
[5-fluoro-2-[2-(2-pyridyloxymethyl)imidazo[1,2-a]pyrimidin-6-yl]phenyl]methyl acetate;
6-[2-(chloromethyl)-4-fluoro-phenyl]-2-[(4-fluorophenoxy)methyl)imidazo[1,2-a]pyrimidine;
6-[4-fluoro-2-(fluoromethyl)phenyl]-2-(2-pyridyloxymethyl)imidazo[1,2-a]pyrimidine; and
6-[4-fluoro-2-(fluoromethyl)phenyl]-2-[(4-fluorophenoxy)methyl]imidazo[1,2-a]pyrimidine.

More specifically, representatives of the compound of Chemical Formula 1 according to the present invention include:
6-(4-fluorophenyl)-2-(pyridin-2-yloxymethyl)imidazo[1,2-a]pyrimidine;
6-[4-fluoro-2-(trifluoromethyl)phenyl]-2-(2-pyridyloxymethyl)imidazo[1,2-a]pyrimidine;
6-(4-fluoro-2-methyl-phenyl)-2-[(5-fluoro-2-pyridyl)oxymethyl)imidazo[1,2-a]pyrimidine;
6-(4-fluorophenyl)-2-[(5-fluoro-2-pyridyl)oxymethyl)imidazo[1,2-a]pyrimidine;
[5-fluoro-2-[2-(2-pyridyloxymethyl)imidazo[1,2-a]pyrimidin-6-yl]phenyl]methanol; and
6-[4-fluoro-2-(fluoromethyl)phenyl]-2-(2-pyridyloxymethyl)imidazo[1,2-a]pyrimidine.

The imidazopyrimidine or imidazotriazine compound of Chemical Formula 1 can be prepared using known compounds or compounds that can be easily prepared therefrom by a person of ordinary skill in the art for compound synthesis. In particular, the method for preparing the compound of Chemical Formula 1 is described in detail in International Patent Publication No. WO 2016/137260 A1, which is incorporated herein by reference. The compound of Chemical Formula 1 may be chemically synthesized by the method described in the above literature, but this is merely to present one exemplary method, and is not intended to limit the scope of the invention as the order of unit operations may be selectively changed as necessary.

The imidazopyrimidine or imidazotriazine compound of Chemical Formula 1 may be used to prevent, alleviate or treat cognitive disorder, or to improve cognitive function.

The imidazopyrimidine or imidazotriazine compound of Chemical Formula 1 may also be applied to the prevention, alleviation or treatment of symptoms of cognitive disorder.

The symptoms of cognitive disorder may arise in a variety of manners including decreased memory, decreased attention, decreased language ability, decreased spatial-temporal ability, decreased reasoning ability, decreased judgment, disorganized thinking, slow thinking, difficulty in understanding, low concentration, loss of problem-solving ability, sloppy memory, difficulty in expressing thoughts, difficulty in integrating thoughts, sensations, and behaviors, or difficulty in eliminating inappropriate thoughts. These symptoms appear as repetition of the same words, repetition of the same question, difficulty in conveying content due to loss, loss of way, and inaccurate memory of names, time or places.

In an embodiment, the imidazopyrimidine or imidazotriazine compound of Chemical Formula 1 may be used to alleviate or treat a disease associated with cognitive disorder.

The disease associated with cognitive disorder may include one or more selected from the group consisting of mild cognitive impairment, Alzheimer's disease, Alzheimer type dementia, presenile dementia, early onset Alzheimer's disease, senile dementia, Lewy body corpuscle dementia, micro-infarct dementia, AIDS-related dementia, HIV-dementia, dementia associated with Lewy bodies, Down's syndrome associated dementia, Pick's disease, recent short-term memory impairment, age-associated cognitive disorder, age-associated memory impairment, drug-associated cognitive disorder, immunodeficiency syndrome-associated cognitive disorder, vascular disease-associated cognitive impairment, cognitive impairment associated with schizophrenia, Parkinson's disease-associated cognitive impairment, cognitive disorders associated with epilepsy, depression-associated cognitive disorder, cognitive disorders associated with bipolar disorder, obsessive compulsive disorder-associated cognitive disorder, post-traumatic stress disorder, attention deficit disorder, attention deficit hyperactivity disorder, and learning deficit disorder.

In an embodiment, the disease associated with cognitive disorder is selected from the group consisting of mild cognitive impairment, Alzheimer's disease, Alzheimer type dementia, presenile dementia, early onset Alzheimer's disease, senile dementia, recent short-term memory impairment, age-associated cognitive disorder, age-associated memory impairment, drug-associated cognitive disorder, and cognitive impairment associated with schizophrenia.

Consequently, the medicament or pharmaceutical composition of the present invention may be used to prevent, alleviate or treat the diseases associated with cognitive disorder, but the scope of the present invention is not limited to the diseases.

In addition, the medicament or pharmaceutical composition of the present invention may be used to prevent, alleviate or treat symptoms of cognitive disorder, and the symptoms include, but are not limited to, decreased attention, decreased language ability, decreased spatial-temporal ability, decreased reasoning ability, or decreased judgment.

The efficacy of the compound of Chemical Formula 1 on cognitive disorder may be verified using a known model. As an animal model related to the prevention and treatment of cognitive disorder, an animal model of which the cognitive ability is decreased by administration of a cognitive inhibitor may be used. As an animal model for improvement of cognitive function, a natural forgetting model in which natural forgetting has occurred over time may be used. Among these, an animal model of which the cognitive ability is decreased by treatment with dizocilpine (MK-801) is widely used as a means to verify drug efficacy for the development of therapeutic agents for cognitive disorder (N.M.W.J de Bruin et al., Neurobiology of learning and memory, 2016, Vol. 133, p. 100-117). A natural forgetting rat model is also used as a means to verify drug efficacy for the development for improvement of cognitive function (M.V. King et al., Neuropharmacology 47 (2004) 195-204*)*.

The dosage of the imidazopyrimidine or imidazotriazine compound of Chemical Formula 1 for the prevention, alleviation or treatment of the disease usually vary depending on the severity of disease, and the body weight and metabolic status of the subject to be treated. A "therapeutically effective amount" for an individual patient means an amount sufficient to achieve the aforementioned pharmacological effect, namely, a therapeutic effect. As the therapeutically effective amount of the compound of Chemical Formula 1, the compound may be contained in the pharmaceutical composition at 0.1 to 500 mg/kg (body weight), preferably 0.5 to 100 mg/kg (body weight) per day when administered to mammals including humans. Such a pharmaceutical composition may be administered one time a day or administered by being divided into two or more times a day.

The compound of the present invention may be administered by conventional methods used for administration of therapeutic agents, such as oral, parenteral, intravenous, intramuscular, subcutaneous or rectal administration.

The medicament or pharmaceutical composition according to an embodiment of the present invention may contain a therapeutically effective amount of a compound selected from the group consisting of the imidazopyrimidine or imidazotriazine compounds of the present invention, pharmaceutically acceptable salts, solvates, and hydrates thereof, and combinations of these.

The pharmaceutically acceptable salt includes both acid or base additional salts and stereochemically isomeric forms thereof. The salt includes any salt that maintains the activity of the parent compound in the object to be administered and does not cause an undesirable effect, and is not particularly limited. Such a salt includes inorganic and organic salts, and may be, for example, salts of acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzenesulfonic acid, benzoic acid, stearic acid, esylic acid, lactic acid, bicarbonic acid, bisulfuric acid, bitartaric acid, oxalic acid, butyric acid, calcium edetate, camsylic acid, carbonic acid, chlorobenzoic acid, citric acid, edetic acid, toluenesulfonic acid, edisylic acid, esylic acid, fumaric acid, gluceptic acid, pamoic acid, gluconic acid, glycollylarsanilic acid, methyl nitric acid, polygalactouronic acid, hexylresorcinoic acid, malonic acid, hydrobamic acid, hydrochloric acid, hydroiodic acid, hydroxy naphthoic acid, isethionic acid, lactobionic acid, mandelic acid, estolic acid, mucic acid, napsylic acid, muconic acid, p-nitromethanesulfonic acid, hexamic acid, pantothenic acid, monohydrogen phosphoric acid, dihydrogen phosphoric acid, salicylic acid, sulfamic acid, sulfanilic acid, methanesulfonic acid, or theoclic acid. In addition, the form of basic salt include, for example, alkali and alkaline earth metal salts such as ammonium salts, lithium salts, sodium salts, potassium salts, magnesium salts and calcium salts; for example, salts with organic bases such as benzathine, N-methyl-D-glucamine, hydrabamine salts; and for example, salts with amino acids such as arginine and lysine. The salt form may also be converted to the free form by being treated with a suitable base or acid. The term "additional salt" includes solvates which the compound of Chemical Formula 1 and salts thereof can form. Such solvates are, for example, hydrates and alcoholates.

The medicament or pharmaceutical composition according to an embodiment of the present invention may be for oral administration or parenteral administration, preferably for oral administration. In the case of parenteral administration, the medicament or pharmaceutical composition may be for parenteral administration such as intravenous injection, subcutaneous injection, intramuscular injection, intraperitoneal injection, endothelial administration, topical administration, intranasal administration, vaginal administration, intrapulmonary administration, or rectal administration. In the case of oral administration, the medicament or pharmaceutical composition of the present invention may be formulated in the form of powders, granules, tablets, pills, sugarcoated tablets, capsules, liquids, gels, syrups, suspensions, wafers, and the like according to the methods known in the art. The pharmaceutical composition according to an embodiment may be formulated to be uncoated or coated with the active agent or to be protected from degradation in the stomach. The composition may be administered by any device capable of transporting the active agent to a target cell. The route of administration may vary depending on the general condition and age of the subject to be treated, the nature of the treatment condition, and the active ingredient selected.

The suitable dosage of the medicament or pharmaceutical composition according to an embodiment of the present invention varies depending on factors such as formulation method, administration mode, and age, weight, sex, pathological condition, food, administration time, administration route, excretion rate and reaction sensitivity of the patient. An ordinarily skilled physician can readily determine and prescribe a dosage effective for the desired treatment or prophylaxis. The pharmaceutical composition according to an embodiment may be administered in one or several doses, for example, may be administered in divided doses from 1 to 4 times a day. The pharmaceutical composition according to an embodiment may contain 0.1 to 500 mg/kg (body weight), preferably 0.5 to 100 mg/kg (body weight) of the compound of Chemical Formula 1.

The medicament or pharmaceutical composition according to an embodiment of the present invention may be prepared in unit dose form by being formulated using a pharmaceutically acceptable carrier and/or filler or by being contained in a multiple-dose container according to a method that can be easily performed by a person of ordinary skill in the art to which the present invention pertains. In this case, the formulation may be in the form of a solution, suspension or emulsion in an oil or aqueous medium, may be in the form of an extract, powder, granule, tablet or capsule, and may additionally contain a dispersant or a stabilizing agent. The pharmaceutical composition may be administered in the form of a suppository, spray, ointment, cream, gel, inhalant or skin patch. The pharmaceutical composition may be prepared for administration to a mammal, more preferably for administration to a human.

The pharmaceutically acceptable carrier may be solid or liquid, and may be one or more selected from a filler, an antioxidant, a buffer, a bacteriostat, a dispersant, an adsorbent, a surfactant, a binder, a preservative, a disintegrant, a sweetening agent, a flavoring agent, a glidant, a release controlling agent, a wetting agent, a stabilizing agent, a suspending agent, and a lubricant. The pharmaceutically acceptable carrier may be selected from saline, sterile water, Ringer's solution, buffered saline, dextrose solution, maltodextrin solution, glycerol, ethanol, and any mixture thereof.

In an embodiment, as a suitable filler, sugars (for example, dextrose, sucrose, maltose and lactose), starch (for example, corn starch), sugar-alcohols (for example, mannitol, sorbitol, maltitol, erythritol and xylitol), starch hydrolysates (for example, dextrin and maltodextrin), cellulose or cellulose derivatives (for example, microcrystalline cellulose), or any mixture thereof may be used, but the filler is not limited thereto.

In an embodiment, as a suitable binder, magnesium aluminum silicate, povidone, copovidone, methylcellulose, hydroxymethylcellulose, hydroxypropylmethylcellulose, hydroxypropylcellulose, hydroxyethylcellulose, gelatin, gum, sucrose, starch paste, or any mixture thereof may be used, but the binder is not limited thereto.

In an embodiment, as a suitable preservative, benzoic acid, sodium benzoate, benzyl alcohol, butylated hydroxyanisole, butylated hydroxytoluene, chlorobutanol, gallate, hydroxybenzoate, EDTA, or any mixture thereof may be used, but the preservative is not limited thereto.

In an embodiment, as a suitable disintegrant, sodium starch glycolate, cross-linked polyvinyl pyrrolidone, cross-linked carboxymethylcellulose, starch, microcrystalline cellulose, or any mixture thereof may be used, but the disintegrant is not limited thereto.

In an embodiment, as a suitable sweetening agent, sucralose, saccharin, sodium or potassium or calcium saccharin, acesulfame potassium or sodium cyclamate, mannitol, fructose, sucrose, maltose, or any mixture thereof may be used, but the sweetening agent is not limited thereto.

In an embodiment, as a suitable glidant, silica, colloidal silicon dioxide, talc and the like may be used, but the glidant is not limited thereto.

In an embodiment, as a suitable lubricant, long chain fatty acids and their salts, for example, magnesium stearate and stearic acid, talc, glyceride wax, or any mixture thereof may be used, but the lubricant is not limited thereto.

The imidazopyrimidine or imidazotriazine compound of Chemical Formula 1 may be used together with an additional drug for prevention, alleviation or treatment of cognitive disorder, or for improving cognitive function.

Accordingly, the medicament or pharmaceutical composition of the present invention may be a combination preparation containing the imidazopyrimidine or imidazotriazine compound of Chemical Formula 1 and such an additional drug as an active ingredient in a therapeutically effective amount.

As such an additional drug, for example, one or more selected from the group consisting of dimethylamylamine, methylphenidate, amphetamine, tacrine, rivastigmine, galantamine, donepezil, memantine, tolcapone, levodopa, atomoxetine, clonidine, pramipexole, guanfacine, and fexofenadine may be used.

Consequently, the medicament or pharmaceutical composition according to an embodiment of the present invention may further contain one or more drugs selected from the group consisting of dimethylamylamine, methylphenidate, amphetamine, tacrine, rivastigmine, galantamine, donepezil, memantine, tolcapone, levodopa, atomoxetine, clonidine, pramipexole, guanfacine, and fexofenadine.

In an embodiment, when the medicament or pharmaceutical composition of the present invention is the above-described combination preparation, the mixed weight ratio (a:b) of the imidazopyrimidine or imidazotriazine compound of Chemical Formula 1 [component (a)] to the additional drug [component (b)] in the combination preparation may be, for example, in a range of 1,000:1 to 1:1,000, or 500:1 to 1:500, or 100:1 to 1:100, or 50:1 to 1:50, or 10:1 to 1:10, but is not limited thereto.

As used herein, the terms "prevent", "preventing", and "prevention" refer to reducing or eliminating the likelihood of contracting a disease.

As used herein, the terms "alleviate", "alleviating", and "alleviation" refer to relieving a disease and/or all or part of its attendant symptoms.

As used herein, the terms "treat", "treating", and "treatment" refer to eliminating a disease and/or all or part of its attendant symptoms.

As used herein, the term "subject" refers to animals, preferably mammals (for example, primates (for example, humans), cattle, sheep, goats, horses, dogs, cats, rabbits, rats, mice, and the like), most preferably humans, that are the objects of treatment, observation or experimentation.

As used herein, the term "therapeutically effective amount" refers to an amount of an active compound or pharmaceutical agent inducing a biological or medical response in a tissue system, animal or human, which is sought by a researcher, veterinarian, physician or other clinician and includes alleviating the symptoms of the disease or disorder to be treated.

As used herein, the term "composition" includes products containing specified ingredients in specified amounts and arbitrary products that result, directly or indirectly, from combinations of specified ingredients in specified amounts.

As used herein, the term "cognitive disorder" refers to a disorder exhibiting atrophy in the cognitive function or cognitive domain of an animal, for example, working memory, attention and arousal, language learning and memory, visual learning and memory, reasoning and problem solving, for example, executive function, task processing speed, and/or social cognition. In particular, it is known that cognitive disorder may exhibit attention deficit, disorganized thinking, slow thinking, difficulty in understanding, low concentration, loss of problem-solving ability, sloppy memory, difficulty in expressing thoughts and/or difficulty in integrating thoughts, sensations and behaviors, or difficulty in eliminating inappropriate thoughts, and may be used interchangeably with the term "cognitive deficit".

### [Advantageous Effects of Invention]

The medicament and pharmaceutical composition according to the present invention can effectively prevent, alleviate or treat cognitive disorder, or improve cognitive function.

### [Brief Description of Drawings]

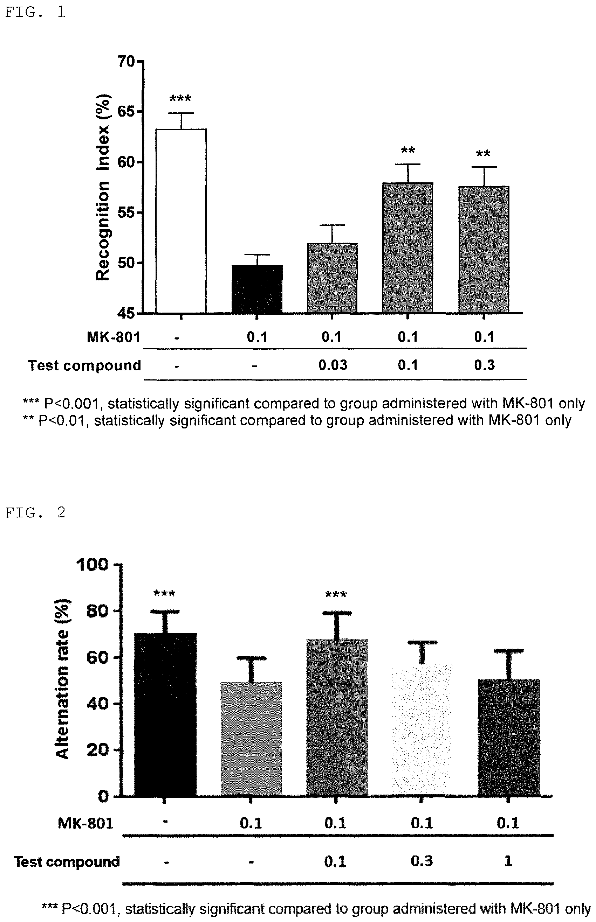

FIG. 1 is results showing the effect of a test compound (0.03, 0.1, and 0.3 mg/kg) on a rat model exhibiting cognitive ability decreased by treatment with dizocilpine in an object recognition test.
FIG. 2 is results showing the effect of a test compound (0.1, 0.3, and 1 mg/kg) on a rat model exhibiting cognitive ability decreased by treatment with dizocilpine in a Y-maze test.

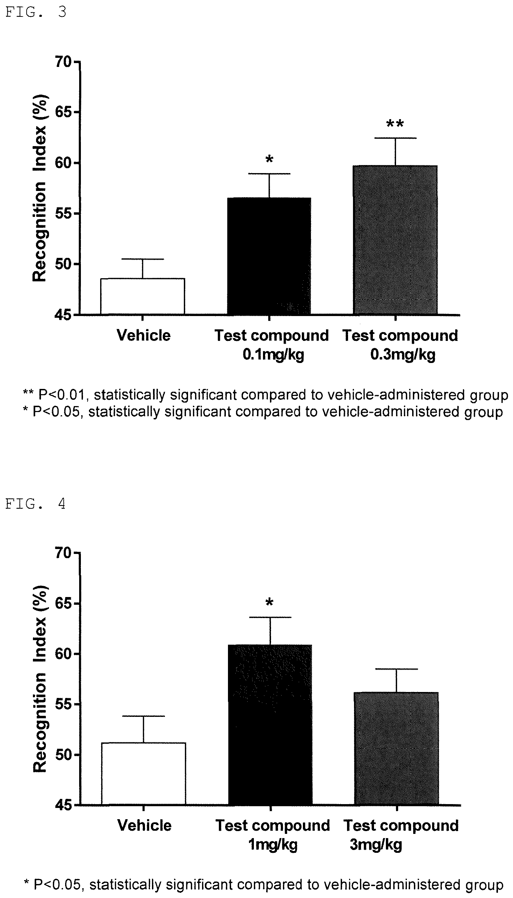

FIG. 3 is results showing the effect of a test compound (0.1 and 0.3 mg/kg) on a natural forgetting rat model in an object recognition test.
FIG.4 is results showing the effect of a test compound (1 and 3 mg/kg) on a natural forgetting rat model in an object recognition test.

### [Description of Embodiments]

Hereinafter, the present invention will be described in more detail through Examples. However, these Examples are only for illustration of one or more embodiments, and the scope of the invention is not limited thereto.

### Preparation Example: Preparation of test compound

According to the method described in Example 113 of International Publication No. WO 2016/137260, 6-[4-fluoro-2-(trifluoromethyl)phenyl]-2-(2-pyridyloxymethyl)imidazo[1,2-a]pyrimidine (test compound) was prepared.

### Example 1: Effect on rat model exhibiting cognitive ability decreased by treatment with dizocilpine in object recognition test

As an animal model of cognitive disorder, an animal model of which the cognitive ability was decreased by administering dizocilpine, a cognitive inhibitor, was used.

### Experimental animal

Male rats (Wistar, 4 weeks old, Orient Bio Co., Ltd.) were purchased and acclimatized for one week or more in an animal cage. The experimental animals were raised in a light-controlled environment (illuminated for 12 hours/nonilluminated for 12 hours) and stored and managed according to the laboratory animal care standards of the Institutional Animal Care and Use Committee (IACUC) in an environment where a temperature of 19°C to 25°C and a relative humidity of 30% to 70% were maintained and water and food could be freely available. The rats were stabilized for one week or more, and then used for an object recognition test. The rats were randomly divided into groups and the test was performed under illumination.

### Drug

To create an animal model inducing symptoms of decreased cognitive ability, the experimental animals were treated with dizocilpine, an N-methyl-D-aspartate (NMDA) receptor inhibitor.

Dizocilpine (purchased from Sigma) was dissolved in physiological saline used as a vehicle to be freshly prepared, and subcutaneously administered at a dose of 0.1 mg/kg in a volume of 1 ml per 1 kg of rat body weight 30 minutes before training. To a control group, only physiological saline was administered.

The test compound was dissolved in 5% DMSO (dimethyl sulfoxide) in 10% Cremophor, and orally administered at doses of 0.03, 0.1, and 0.3 mg/kg in a volume of 1 ml per 1 kg of rat body weight one hour before training. To a control group, only vehicle (5% DMSO in 10% Cremophor) was administered.

### Object recognition test

Each rat was handled by the experimenter for 3 minutes or more, and box acclimation for 3 minutes was performed two times in an empty box before the experiment. After administration of vehicle or test compound intraperitoneally at a dose of 1 mg/kg one hour before training and of vehicle or dizocilpine subcutaneously 30 minutes before training, the rats were boxed for training. Training was conducted by exposing the rats in a box in which two identical plastic cylinders or stainless square pyramids were placed. One hour after training, the test was performed in a box in which one plastic cylinder and one stainless square pyramid was placed. During the test, the time during which the rat explored each object was measured. The test results were analyzed using a stainless square pyramid as a novel object in the case of rats trained in a box in which two plastic cylinders were placed and a plastic cylinder as a novel object in the case of rats trained in a box in which two stainless steel pyramids were placed.

### Statistical analysis of experimental results

The recognition index was calculated based on the time during which the object was explored, the data on the recognition index between groups was analyzed by one-way analysis of variance (ANOVA) and Dunnett's multiple comparison test, and then the effect was defined as significant when p < 0.05. All results were expressed as mean ± SEM.

### Test results

The recognition index of the negative control group treated with only dizocilpine was 49.77 ± 1.03 seconds, and a significant decrease in cognitive function compared to that of the vehicle group, which is a positive control group, having a recognition index of 63.29 ± 1.56 seconds was observed (P < 0.001) . When dizocilpine and the test compound were administered together, the recognition indexes of the 0.1 mg/kg administration group and the 0.3 mg/kg administration group were 57.95 ± 1.8 seconds and 57.62 ± 1.89 seconds, respectively, and significant cognitive function recovery compared to the negative control group exhibiting decreased cognitive function was observed (both P < 0.01).

The analysis results of recognition index of a rat model exhibiting cognitive ability decreased by treatment with dizocilpine in an object recognition test using a test compound are summarized in Table 1.

**[Table 1] Analysis results of recognition index of rat model exhibiting cognitive ability decreased by treatment with dizocilpine in object recognition test using test compound**

| Treated drug | Dosage | | Recognition index¹⁾ (mean ± SEM) |
|---|---|---|---|
| | Dizocilpine (mg/kg, sc²⁾) | Test compound (mg/kg, po³⁾) | |
| Vehicle | N_{T} ⁴⁾ | NT | 63.29±1.56 |
| Dizocilpine | 0.1 | NT | 49.77±1.03 |
| Dizocilpine and test compound | 0.1 | 0.03 | 51.95±1.79 |
| Dizocilpine and test compound | 0.1 | 0.1 | 57.95±1.8 |
| Dizocilpine and test compound | 0.1 | 0.3 | 57.62±1.89 |

| | | | |
|---|---|---|---|
| ¹⁾ Recognition index = exploring time of novel object/total exploring time (identical object + novel object) × 100 ²⁾ sc = Subcutaneously ³⁾ po = Oral administration (per os) ⁴⁾ NT = Not treated | | | |

From this, a synergistic increase in recovery effect on the impairment of cognitive function has been confirmed as a result of administration of the test compound.

### Example 2: Effect on rat model exhibiting cognitive ability decreased by treatment with dizocilpine in Y-maze test

### Experimental animal

Male rats (Wistar, 4 weeks old, Orient Bio Co., Ltd.) were purchased and acclimatized for one week or more in an animal cage. The experimental animals were raised in a light-controlled environment (illuminated for 12 hours/nonilluminated for 12 hours) and stored and managed according to the laboratory animal care standards of the Institutional Animal Care and Use Committee in an environment where a temperature of 19°C to 25°C and a relative humidity of 30% to 70% were maintained and water and food could be freely available. The rats were stabilized for one week or more, and then used for a Y-maze test. The rats were randomly divided into groups and the test was performed under illumination.

### Drug

To create an animal model inducing symptoms of decreased cognitive ability, experimental animals were treated with dizocilpine, an NMDA receptor inhibitor.

Dizocilpine (purchased from Sigma) was dissolved in physiological saline used as a vehicle to be freshly prepared, and subcutaneously administered at a dose of 0.1 mg/kg in a volume of 1 ml per 1 kg of rat body weight 30 minutes before training. To a control group, only physiological saline was administered.

The test compound was dissolved in 5% DMSO in 9.5% Cremophor, and orally administered at doses of 0.1, 0.3, and 1 mg/kg in a volume of 5 ml per 1 kg of rat body weight one hour before training. To a control group, only vehicle (5% DMSO in 9.5% Cremophor) was administered.

### Y-maze test

The rats were handled by the experimenter for 3 minutes for two days, and a Y-maze test was performed the next day. After administration of vehicle or test compound intraperitoneally one hour before test and of vehicle or dizocilpine subcutaneously 30 minutes before test, the Y-maze test was performed for 5 minutes by placing the rat at the end of one arm in the Y-maze and allowing the rat to move freely. The three arms positioned at the same angle in the Y-maze had the same length of 45 cm, the same width of 10 cm, and the same height of 20 cm. A case where a rat visited three different arms in succession was defined as an alternation behavior.

### Statistical analysis of experimental results

The alternation rate was determined by identifying the alternation behavior of the rat based on the visit record to each arm in the Y-maze test, the data on the alternation rate between groups was analyzed by one-way ANOVA and Dunnett's multiple comparison test, and then the effect was defined as significant when p < 0.05. All results were expressed as mean ± SEM.

### Test results

The alternation rate of the negative control group treated with only dizocilpine was 49.2 ± 5.3 seconds, and a significant decrease in cognitive function compared to the vehicle group, which is a positive control group, having an alternation rate of 70.1 ± 2.8 seconds was observed (P < 0.001). When dizocilpine and the test compound were administered together, the alternation rate of the 0.1 mg/kg administration group was 67.3 ± 3.5 seconds, and significant cognitive function recovery compared to the negative control group exhibiting decreased cognitive function was observed (P < 0.001).

The alternation rate of the 0.3 mg/kg administration group of the test compound was 57.3 ± 5.7 seconds, and a tendency toward the recovery of cognitive function was observed compared to the control group exhibiting decreased cognitive function.

The analysis results of alternation rate of a rat model exhibiting cognitive ability decreased by treatment with dizocilpine in a Y-maze test using a test compound are summarized in Table 2.

**[Table 2] Analysis results of alternation rate of rat model exhibiting cognitive ability decreased by treatment with dizocilpine in Y-maze test using test compound**

| Treated drug | Dosage | | Alternation rate¹⁾ (mean ± SEM) |
|---|---|---|---|
| | Dizocilpine (mg/kg, sc²⁾) | Test compound (mg/kg, po³⁾) | |
| Vehicle | NT ⁴⁾ | NT | 70.1±2.8 |
| Dizocilpine | 0.1 | NT | 49.2±5.3 |
| Dizocilpine and test compound | 0.1 | 0.1 | 67.3±3.5 |
| Dizocilpine and test compound | 0.1 | 0.3 | 57.3±5.7 |
| Dizocilpine and test compound | 0.1 | 1 | 49.9±7.2 |

| | | | |
|---|---|---|---|
| ¹⁾ Alternation rate = number of actual alternation behaviors/maximum possible number of alternation behaviors × 100 [maximum possible number of alternation behaviors = number of total entries - 2] ²⁾ sc = Subcutaneously ³⁾ po = Oral administration (per os) ⁴⁾ NT = Not treated | | | |

From this, a synergistic increase in recovery effect on the impairment of cognitive function has been confirmed as a result of administration of the test compound.

### Example 3: Effect on rat model undergone natural forgetting in object recognition test

As an animal model of cognitive disorder, a natural forgetting model in which natural forgetting occurred over time was used.

### Experimental animal

Male rats (Wistar, 4 weeks old, Orient Bio Co., Ltd.) were purchased and acclimatized for one week or more in an animal cage. The experimental animals were raised in a light-controlled environment (illuminated for 12 hours/nonilluminated for 12 hours) and stored and managed according to the laboratory animal care standards of the Institutional Animal Care and Use Committee in an environment where a temperature of 19°C to 25°C and a relative humidity of 30% to 70% were maintained and water and food could be freely available. The rats were stabilized for one week or more, and then used for an object recognition test. The rats were randomly divided into groups and the test was performed under illumination.

### Drug

The test compound was dissolved in 5% DMSO in 10% Cremophor, and orally administered at a dose of 1 mg/kg one hour before training. To a control group, only vehicle (5% DMSO in 10% Cremophor) was administered.

### Object recognition test

Each rat was handled by the experimenter for 3 minutes or more, and box acclimation for 3 minutes was performed two times in an empty box before the experiment. After administration of vehicle or test compound intraperitoneally one hour before training at a dose of 1 mg/kg, the rats were boxed for training. Training was conducted by exposing the rats in a box in which two identical plastic cylinders or stainless square pyramids were placed. The test was performed 24 hours after training in a box in which one plastic cylinder and one stainless square pyramid was placed. During the test, the time during which the rat explored each object was measured. The test results were analyzed using a stainless square pyramid as a novel object in the case of rats trained in a box in which two plastic cylinders were placed and a plastic cylinder as a novel object in the case of rats trained in a box in which two stainless steel pyramids were placed.

### Statistical analysis of experimental results

The recognition index was calculated based on the time during which the object was explored, the data on the recognition index between groups was analyzed by one-way ANOVA and Dunnett's multiple comparison test, and then the effect was defined as significant when p < 0.05. All results were expressed as mean ± SEM.

### Test results

The experiment was performed two times according to the dose of the test compound. In the first experiment, the recognition index in the vehicle group, which is a control group, was 48.61 ± 1.87 seconds and did not statistically differ from 50 which was the recognition index when the group was not able to distinguish the identical object from a novel object but explored for exactly the same time, and it was thus confirmed that sufficient natural forgetting occurred for 24 hours.

When the test compound was administered at 0.1 mg/kg and 0.3 mg/kg, the recognition indexes were 56.57 ± 2.37 seconds and 59.77 ± 2.64 seconds, respectively, and were significantly different from that in the control group undergone natural forgetting (P < 0.05, P < 0.01, respectively).

The analysis results of recognition index of a rat model undergone natural forgetting in an object recognition test using a test compound (0.1 mg/kg and 0.3 mg/kg) are summarized in Table 3.

**[Table 3] Analysis results of recognition index of rat model undergone natural forgetting in object recognition test using test compound (0.1 mg/kg and 0.3 mg/kg)**

| Treated drug | Dosage of test compound (mg/kg, po²⁾) | Recognition index¹⁾ (mean ± SEM) |
|---|---|---|
| Vehicle | NT ³⁾ | 48.61±1.87 |
| Test compound | 0.1 | 56.57±2.37 |
| Test compound | 0.3 | 59.77±2.64 |

| | | |
|---|---|---|
| ¹⁾ Recognition index = exploring time of novel object/total exploring time(identical object + novel object) × 100 ²⁾ po = Oral administration (per os) ³⁾ NT = Not treated | | |

In the second experiment as well, the recognition index in the vehicle group, which is a control group, was 51.20 ± 2.61 seconds, and did not statistically differ from 50 which was the recognition index when the group was not able to distinguish the identical object from a novel object but explored for exactly the same time, and it has been thus confirmed that sufficient natural forgetting occurred for 24 hours.

When the test compound was administered at 1 mg/kg, the recognition index was 60.91 ± 2.72 seconds and was significantly different from that in the control group undergone natural forgetting (P < 0.01). The recognition index in the 3 mg/kg administration group of the test compound was 56.20 ± 2.27 seconds, and this indicates that natural forgetting is alleviated compared to the control group undergone natural forgetting.

The analysis results of recognition index of a rat model undergone natural forgetting in an object recognition test using a test compound (1 mg/kg and 3 mg/kg) are summarized in Table 4.

**[Table 4] Analysis results of recognition index of rat model undergone natural forgetting in object recognition test using test compound (1 mg/kg and 3 mg/kg)**

| Treated drug | Dosage of test compound (mg/kg, po²⁾) | Recognition index¹⁾ (mean ± SEM) |
|---|---|---|
| Vehicle | NT ³⁾ | 51.20±2.61 |
| Test compound | 1 | 60.91±2.72 |
| Treated drug | 3 | 56.20±2.27 |

| | | |
|---|---|---|
| ¹⁾ Recognition index = exploring time of novel object/total exploring time (identical object + novel object) × 100 ²⁾ po = Oral administration (per os) ³⁾ NT = Not treated | | |

From the experimental results in such a natural forgetting model, the natural forgetting alleviating effect of the test compound through enhancement of cognitive function in a normal cognitive state has been confirmed.

## Claims

1. A medicament for prevention, alleviation or treatment of cognitive disorder, or for improving cognitive function, which comprises a therapeutically effective amount of an imidazopyrimidine or imidazotriazine compound of Chemical Formula 1, or a pharmaceutically acceptable salt, solvate or hydrate thereof: 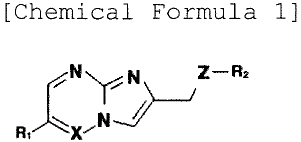 in Chemical Formula 1,
X is CH or N;
Z is O or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl, C₁-C₅ alkyl-C(O)O-C₁-C₅ alkyl, a 5- or 6-membered heterocycloalkyl-Ci-C₅ alkyl having 1 to 3 heteroatoms selected from N, O and S, and di(C₁-C₅ alkyl) amino-C₁-C₅ alkyl; or 5- to 12-membered unsaturated heterocyclyl having 1 to 5 heteroatoms selected from N, O and S, unsubstituted or substituted with one or more substituents selected from halo, hydroxy, C₁-C₅ alkyl, C₁-C₅ alkoxy, and halo-Ci-Cs alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

2. The medicament according to claim 1, wherein R₁ is phenyl unsubstituted or substituted with 1 to 3 substituents selected from halo, hydroxy, C₁-C₅ alkyl, C₁-C₅ alkoxy, C₁-C₅ alkylthio, amino, di (C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl and C₁-C₅ alkyl-C(O)O-C₁-C₅ alkyl; or 5- to 10-membered unsaturated heterocyclyl having 1 to 3 heteroatoms selected from N, O and S, unsubstituted or substituted with 1 to 3 substituents selected from halo, hydroxy, C₁-C₅ alkyl, C₁-C₅ alkoxy and halo-C₁-C₅ alkyl.

3. The medicament according to claim 1, wherein R₂ is phenyl unsubstituted or substituted with 1 to 5 substituents selected from halo, deuterium, hydroxy and C₁-C₅ alkyl; or 5- or 6-membered heteroaryl having 1 to 3 heteroatoms selected from N, 0 and S, unsubstituted or substituted with 1 to 3 substituents selected from halo and C₁-C₅ alkyl.

4. The medicament according to claim 1, wherein
X is CH or N;
Z is 0;
R₁ is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl or C₁-C₅ alkyl-C(O)O-C₁-C₅ alkyl; or 5- to 9-membered unsaturated heterocyclyl having 1 or 2 heteroatoms selected from N, O or S, unsubstituted or substituted with 1 or 2 substituents selected from halo, hydroxy, C₁-C₅ alkyl, C₁-C₅ alkoxy and halo-C₁-C₅ alkyl; and
R₂ is phenyl unsubstituted or substituted with 1 to 5 substituents selected from halo, deuterium, hydroxy and C₁-C₅ alkyl; or 6-membered heteroaryl having 1 or 2 nitrogen atoms, unsubstituted or substituted with 1 or 2 substituents selected from halo and C₁-C₅ alkyl.

5. The medicament according to claim 1, wherein
R₁ is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl and C₁-C₅ alkyl-C(O)O-C₁-C₅ alkyl; 1,3-benzodioxolyl unsubstituted or substituted with 1 or 2 halo; or pyridyl or pyrimidinyl unsubstituted or substituted with 1 or 2 substituents selected from halo, hydroxy, C₁-C₅ alkyl, C₁-C₅ alkoxy and halo-C₁-C₅ alkyl, and
R₂ is phenyl unsubstituted or substituted with 1 to 5 substituents selected from halo, deuterium, hydroxy and C₁-C₅ alkyl; or pyridyl unsubstituted or substituted with 1 or 2 substituents selected from halo and C₁-C₅ alkyl.

6. The medicament according to claim 1, wherein
X is CH;
Z is 0;
R₁ is phenyl unsubstituted or substituted with 1 to 3 substituents selected from halo, hydroxy, C₁-C₅ alkyl, C₁-C₅ alkoxy, C₁-C₅ alkylthio, amino, halo-C₁-C₅ alkyl, hydroxy-C₁-C₅ alkyl and C₁-C₅ alkoxy-C₁-C₅ alkyl; and
R₂ is pyridyl unsubstituted or substituted with 1 or 2 substituents selected from halo and C₁-C₅ alkyl.

7. The medicament according to claim 1, wherein the imidazopyrimidine or imidazotriazine compound of Chemical Formula 1 is selected from the following compounds:
6-(4-fluorophenyl)-2-(pyridin-2-yloxymethyl)imidazo[1,2-a]pyrimidine;
6-[4-fluoro-2-(trifluoromethyl)phenyl]-2-(2-pyridyloxymethyl)imidazo[1,2-a]pyrimidine;
6-(4-fluoro-2-methyl-phenyl)-2-[(5-fluoro-2-pyridyl)oxymethyl)imidazo[1,2-a]pyrimidine;
6-(4-fluorophenyl)-2-[(5-fluoro-2-pyridyl)oxymethyl)imidazo[1,2-a]pyrimidine;
[5-fluoro-2-[2-(2-pyridyloxymethyl)imidazo[1,2-a]pyrimidin-6-yl]phenyl]methanol; and
6-[4-fluoro-2-(fluoromethyl)phenyl]-2-(2-pyridyloxymethyl)imidazo[1,2-a]pyrimidine.

8. The medicament according to claim 1, which is used for preventing, alleviating or treating symptoms of cognitive disorder.

9. The medicament according to claim 8, wherein the symptoms of cognitive disorder are decreased attention, decreased language ability, decreased spatial-temporal ability, decreased reasoning ability, or decreased judgment.

10. The medicament according to claim 1, which is used for alleviating or treating a disease associated with cognitive disorder.

11. The medicament according to claim 10, wherein the disease associated with cognitive disorder is selected from the group consisting of mild cognitive impairment, Alzheimer's disease, Alzheimer type dementia, presenile dementia, early onset Alzheimer's disease, senile dementia, Lewy body corpuscle dementia, micro-infarct dementia, AIDS-related dementia, HIV-dementia, dementia associated with Lewy bodies, Down's syndrome associated dementia, Pick's disease, recent short-term memory impairment, age-associated cognitive disorder, age-associated memory impairment, drug-associated cognitive disorder, immunodeficiency syndrome-associated cognitive disorder, vascular disease-associated cognitive impairment, cognitive impairment associated with schizophrenia, Parkinson's disease-associated cognitive impairment, cognitive disorders associated with epilepsy, depression-associated cognitive disorder, cognitive disorders associated with bipolar disorder, obsessive compulsive disorder-associated cognitive disorder, post-traumatic stress disorder, attention deficit disorder, attention deficit hyperactivity disorder, and learning deficit disorder.

12. The medicament according to claim 11, wherein the disease associated with cognitive disorder is selected from the group consisting of mild cognitive impairment, Alzheimer's disease, Alzheimer type dementia, presenile dementia, early onset Alzheimer's disease, senile dementia, recent short-term memory impairment, age-associated cognitive disorder, age-associated memory impairment, drug-associated cognitive disorder, and cognitive impairment associated with schizophrenia.

13. The medicament according to claim 1, which is prepared for administration to a mammal.

14. The medicament according to claim 1, comprising 0.1 to 500 mg/kg (body weight) of the imidazopyrimidine or imidazotriazine compound of Chemical Formula 1.

15. The medicament according to claim 1, which is for oral administration or for parenteral administration selected from the group consisting of intravenous injection, subcutaneous injection, intramuscular injection, intraperitoneal injection, endothelial administration, topical administration, intranasal administration, vaginal administration, intrapulmonary administration and rectal administration.

16. The medicament according to claim 1, further comprising one or more drugs selected from the group consisting of dimethylamylamine, methylphenidate, amphetamine, tacrine, rivastigmine, galantamine, donepezil, memantine, tolcapone, levodopa, atomoxetine, clonidine, pramipexole, guanfacine, and fexofenadine.

17. A pharmaceutical composition for prevention, alleviation or treatment of cognitive disorder, or for improving cognitive function, which comprises a therapeutically effective amount of an imidazopyrimidine or imidazotriazine compound of Chemical Formula 1, or a pharmaceutically acceptable salt, solvate or hydrate thereof, and additionally one or more pharmaceutically acceptable carriers: 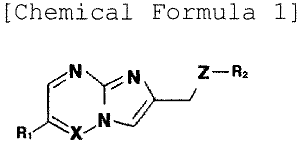 in Chemical Formula 1,
X is CH or N;
Z is O or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-Ci-Cs alkyl, hydroxy-C₁-C₅ alkyl, C₁-C₅ alkoxy-C₁-C₅ alkyl, carbamoyloxy-Ci-Cs alkyl, C₁-C₅ alkyl-C(O)O-C₁-C₅ alkyl, a 5- or 6-membered heterocycloalkyl-C₁-C₅ alkyl having 1 to 3 heteroatoms selected from N, O and S, and di(C₁-C₅ alkyl) amino-C₁-C₅ alkyl; or 5- to 12-membered unsaturated heterocyclyl having 1 to 5 heteroatoms selected from N, O and S, unsubstituted or substituted with one or more substituents selected from halo, hydroxy, C₁-C₅ alkyl, C₁-C₅ alkoxy, and halo-C₁-C₅ alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

18. The pharmaceutical composition according to claim 17, which is used for preventing, alleviating or treating symptoms of cognitive disorder.

19. The pharmaceutical composition according to claim 18, wherein the symptoms of cognitive disorder are decreased attention, decreased language ability, decreased spatial-temporal ability, decreased reasoning ability, or decreased judgment.

20. The pharmaceutical composition according to claim 17, which is used for alleviating or treating a disease associated with cognitive disorder.

21. The pharmaceutical composition according to claim 20, wherein the disease associated with cognitive disorder is selected from the group consisting of mild cognitive impairment, Alzheimer's disease, Alzheimer type dementia, presenile dementia, early onset Alzheimer's disease, senile dementia, Lewy body corpuscle dementia, micro-infarct dementia, AIDS-related dementia, HIV-dementia, dementia associated with Lewy bodies, Down's syndrome associated dementia, Pick's disease, recent short-term memory impairment, age-associated cognitive disorder, age-associated memory impairment, drug-associated cognitive disorder, immunodeficiency syndrome-associated cognitive disorder, vascular disease-associated cognitive impairment, cognitive impairment associated with schizophrenia, Parkinson's disease-associated cognitive impairment, cognitive disorders associated with epilepsy, depression-associated cognitive disorder, cognitive disorders associated with bipolar disorder, obsessive compulsive disorder-associated cognitive disorder, post-traumatic stress disorder, attention deficit disorder, attention deficit hyperactivity disorder, and learning deficit disorder.

22. The pharmaceutical composition according to claim 21, wherein the disease associated with cognitive disorder is selected from the group consisting of mild cognitive impairment, Alzheimer's disease, Alzheimer type dementia, presenile dementia, early onset Alzheimer's disease, senile dementia, recent short-term memory impairment, age-associated cognitive disorder, age-associated memory impairment, drug-associated cognitive disorder, and cognitive impairment associated with schizophrenia.

23. The pharmaceutical composition according to claim 17, which is prepared for administration to a mammal.

24. The pharmaceutical composition according to claim 17, comprising 0.1 to 500 mg/kg (body weight) of the imidazopyrimidine or imidazotriazine compound of Chemical Formula 1.

25. The pharmaceutical composition according to claim 17, which is for oral administration or for parenteral administration selected from the group consisting of intravenous injection, subcutaneous injection, intramuscular injection, intraperitoneal injection, endothelial administration, topical administration, intranasal administration, vaginal administration, intrapulmonary administration and rectal administration.

26. The pharmaceutical composition according to claim 17, wherein the pharmaceutically acceptable carrier is one or more selected from a filler, an antioxidant, a buffer, a bacteriostat, a dispersant, an adsorbent, a surfactant, a binder, a preservative, a disintegrant, a sweetening agent, a flavoring agent, a glidant, a release controlling agent, a wetting agent, a stabilizing agent, a suspending agent and a lubricant.

27. The pharmaceutical composition according to claim 17, further comprising one or more drugs selected from the group consisting of dimethylamylamine, methylphenidate, amphetamine, tacrine, rivastigmine, galantamine, donepezil, memantine, tolcapone, levodopa, atomoxetine, clonidine, pramipexole, guanfacine, and fexofenadine.

28. A method for prevention, alleviation or treatment of cognitive disorder, or for improving cognitive function in a subject, which comprises administering to the subject a therapeutically effective amount of an imidazopyrimidine or imidazotriazine compound of Chemical Formula 1, or a pharmaceutically acceptable salt, solvate or hydrate thereof: 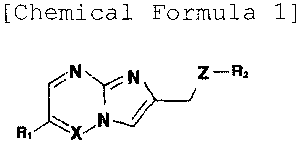 in Chemical Formula 1,
X is CH or N;
Z is O or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-Ci-Cs alkyl, C₁-C₅ alkoxy-C₁-C₅ alkyl, carbamoyloxy-C₁-C₅ alkyl, C₁-C₅ alkyl-C(O)O-C₁-C₅ alkyl, a 5- or 6-membered heterocycloalkyl-Ci-C₅ alkyl having 1 to 3 heteroatoms selected from N, O and S, and di(C₁-C₅ alkyl) amino-C₁-C₅ alkyl; or 5- to 12-membered unsaturated heterocyclyl having 1 to 5 heteroatoms selected from N, 0 and S, unsubstituted or substituted with one or more substituents selected from halo, hydroxy, C₁-C₅ alkyl, C₁-C₅ alkoxy, and halo-C₁-C₅ alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

29. The method according to claim 28, which is for preventing, alleviating or treating symptoms of cognitive disorder.

30. The method according to claim 29, wherein the symptoms of cognitive disorder are decreased attention, decreased language ability, decreased spatial-temporal ability, decreased reasoning ability, or decreased judgment.

31. The method according to claim 28, which is for alleviating or treating a disease associated with cognitive disorder.

32. The method according to claim 31, wherein the disease associated with cognitive disorder is selected from the group consisting of mild cognitive impairment, Alzheimer's disease, Alzheimer type dementia, presenile dementia, early onset Alzheimer's disease, senile dementia, Lewy body corpuscle dementia, micro-infarct dementia, AIDS-related dementia, HIV-dementia, dementia associated with Lewy bodies, Down's syndrome associated dementia, Pick's disease, recent short-term memory impairment, age-associated cognitive disorder, age-associated memory impairment, drug-associated cognitive disorder, immunodeficiency syndrome-associated cognitive disorder, vascular disease-associated cognitive impairment, cognitive impairment associated with schizophrenia, Parkinson's disease-associated cognitive impairment, cognitive disorders associated with epilepsy, depression-associated cognitive disorder, cognitive disorders associated with bipolar disorder, obsessive compulsive disorder-associated cognitive disorder, post-traumatic stress disorder, attention deficit disorder, attention deficit hyperactivity disorder, and learning deficit disorder.

33. The method according to claim 32, wherein the disease associated with cognitive disorder is selected from the group consisting of mild cognitive impairment, Alzheimer's disease, Alzheimer type dementia, presenile dementia, early onset Alzheimer's disease, senile dementia, recent short-term memory impairment, age-associated cognitive disorder, age-associated memory impairment, drug-associated cognitive disorder, and cognitive impairment associated with schizophrenia.

34. The method according to claim 28, wherein the subject is a mammal.

35. The method according to claim 28, wherein 0.1 to 500 mg/kg (body weight) of the imidazopyrimidine or imidazotriazine compound of Chemical Formula 1 is administered.

36. The method according to claim 28, wherein one or more drugs selected from the group consisting of dimethylamylamine, methylphenidate, amphetamine, tacrine, rivastigmine, galantamine, donepezil, memantine, tolcapone, levodopa, atomoxetine, clonidine, pramipexole, guanfacine, and fexofenadine are additionally administered.

37. Use of an imidazopyrimidine or imidazotriazine compound of Chemical Formula 1, or a pharmaceutically acceptable salt, solvate or hydrate thereof for prevention, alleviation or treatment of cognitive disorder, or for improving cognitive function: 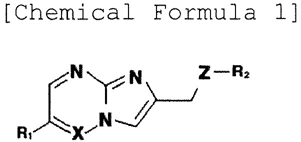 in Chemical Formula 1,
X is CH or N;
Z is 0 or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di (C₁-C₅ alkyl) amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl, C₁-C₅ alkyl-C(O)O-C₁-C₅ alkyl, a 5- or 6-membered heterocycloalkyl-C₁-C₅ alkyl having 1 to 3 heteroatoms selected from N, 0 or S, or di(C₁-C₅ alkyl)amino-C₁-C₅ alkyl; or 5- to 12-membered unsaturated heterocyclyl having 1 to 5 heteroatoms selected from N, O or S, unsubstituted or substituted with one or more substituents selected from halo, hydroxy, C₁-C₅ alkyl, C₁-C₅ alkoxy, or halo-C₁-C₅ alkyl;
R₂ is C₆-C₁₂ aryl unsubstituted or substituted with one or more substituents selected from halo, deuterium, hydroxy or C₁-C₅ alkyl; or 5- to 12-membered unsaturated heterocyclyl having 1 to 3 heteroatoms selected from N, O or S, unsubstituted or substituted with one or more substituents selected from halo or C₁-C₅ alkyl.

38. The use according to claim 37, which is for preventing, alleviating or treating symptoms of cognitive disorder.

39. The use according to claim 38, wherein the symptoms of cognitive disorder are decreased attention, decreased language ability, decreased spatial-temporal ability, decreased reasoning ability, or decreased judgment.

40. The use according to claim 37, which is for alleviating or treating a disease associated with cognitive disorder.

41. The use according to claim 40, wherein the disease associated with cognitive disorder is selected from the group consisting of mild cognitive impairment, Alzheimer's disease, Alzheimer type dementia, presenile dementia, early onset Alzheimer's disease, senile dementia, Lewy body corpuscle dementia, micro-infarct dementia, AIDS-related dementia, HIV-dementia, dementia associated with Lewy bodies, Down's syndrome associated dementia, Pick's disease, recent short-term memory impairment, age-associated cognitive disorder, age-associated memory impairment, drug-associated cognitive disorder, immunodeficiency syndrome-associated cognitive disorder, vascular disease-associated cognitive impairment, cognitive impairment associated with schizophrenia, Parkinson's disease-associated cognitive impairment, cognitive disorders associated with epilepsy, depression-associated cognitive disorder, cognitive disorders associated with bipolar disorder, obsessive compulsive disorder-associated cognitive disorder, post-traumatic stress disorder, attention deficit disorder, attention deficit hyperactivity disorder, and learning deficit disorder.

42. The use according to claim 41, wherein the disease associated with cognitive disorder is selected from the group consisting of mild cognitive impairment, Alzheimer's disease, Alzheimer type dementia, presenile dementia, early onset Alzheimer's disease, senile dementia, recent short-term memory impairment, age-associated cognitive disorder, age-associated memory impairment, drug-associated cognitive disorder, and cognitive impairment associated with schizophrenia.

43. The use according to claim 37, which is for administration to a mammal.

44. The use according to claim 37, wherein 0.1 to 500 mg/kg (body weight) of the imidazopyrimidine or imidazotriazine compound of Chemical Formula 1 is used.

45. The use according to claim 37, wherein one or more drugs selected from the group consisting of dimethylamylamine, methylphenidate, amphetamine, tacrine, rivastigmine, galantamine, donepezil, memantine, tolcapone, levodopa, atomoxetine, clonidine, pramipexole, guanfacine, and fexofenadine are additionally used.
